# EUROPEAN PATENT APPLICATION

(11) **EP 4 310 719 A1**
(43) Date of publication of application: **24.01.2024**
(21) Application number: 22771408.6
(22) Date of filing: 15.03.2022
(51) Int. Cl.: G06F 30/27, G01N 33/20

(54) **MATERIAL CHARACTERISTICS PREDICTION METHOD AND MODEL GENERATION METHOD**

(30) Priority: 17.03.2021 JP 2021043191
(71) Applicant: Resonac Corporation, Tokyo 105-8518 (JP)
(72) Inventor: TAKEMOTO, Shimpei, Tokyo 105-8518 (JP); OKUNO, Yoshishige, Tokyo 105-8518 (JP); KANESHITA, Takeshi, Tokyo 105-8518 (JP)
(74) Representative: Strehl Schübel-Hopf & Partner
(86) International application number: PCT/JP2022/011453
(87) International publication number: WO 2022/196663

(57) **Abstract**

Model setting step sets a trained model acquired by machine learning of a correspondence relationship between an explanatory variable including information related to a material composition or a manufacturing condition of a target material, and an objective variable including information related to the material characteristics of the target material, and prediction step inputs an explanatory variable related to a target material whose material characteristics is to be predicted to the trained model set in the model setting step, and outputs an objective variable related to information of the explanatory variable, so as to predict the material characteristics of the target material to be predicted based on the objective variable. The explanatory variable includes a material characteristics evaluation temperature that is a temperature at a time of measurement of the material characteristics included in the objective variable, and an evaluation temperature holding time that is a time during which the material characteristics evaluation temperature is held until the measurement of the material characteristics.

## Description

### TECHNICAL FIELD

The present disclosure relates to material characteristics prediction methods, material characteristics prediction programs, material characteristics prediction devices, model generation methods, model generation programs, and model generation devices.

### BACKGROUND ART

Conventionally, when designing a material composed of a plurality of compositions or a material manufactured by a combination of a plurality of manufacturing conditions, an optimum solution capable of achieving desired material characteristics is obtained, by repeating trial manufacture while adjusting the composition of the material and the manufacturing conditions based on experience of a material developer. However, in many cases, the trial manufacture based on the experience of the material developer needs to be repeated until the optimum design is obtained, thereby requiring time and effort. In addition, in many cases, a condition search is locally performed in a vicinity of a design condition performed by the material developer in the past, and is unsuited for a global search for an optimum design condition.

Hence, in recent years, a material may be designed using a database of past trial manufacture and evaluation, or material characteristics may be predicted by applying machine learning to a database.

In addition, due to increased needs for weight reduction in automobiles or the like, there are demands to develop aluminum alloys or the like that can exhibit high strength even at high temperatures of 150°C or higher, for example. In general, the material characteristics tend to vary according to an ambient temperature at which the characteristics are measured. For this reason, it is desirable to be able to predict the material characteristics under a temperature condition other than a room temperature, such as a high temperature higher than the room temperature, a low temperature lower than the room temperature, or the like, using a prediction model based on the machine learning.

However, a prediction accuracy of the material characteristics by the machine learning greatly depends on the number of measurement data. In general, the number of measurement data of the material characteristics at the high temperature, requiring time and effort for the measurement, is smaller than the number of measurement data of the material characteristics at the room temperature, and it is difficult to increase the prediction accuracy of a high-temperature characteristics prediction model. That is, it is difficult to build a highly reliable prediction model for designing the material exhibiting a high strength at the high temperature. The same holds true for the low temperature.

Methods for improving the prediction accuracy even when the number of data for generating the prediction model is small, include a method of separately building a prediction model for each of a plurality of conditions having different output characteristics, a method of building a prediction model by transfer learning in which a trained model that is built through training by proxy output characteristics having a large number of data is retrained by target output characteristics having an insufficient number of data, a method of building a prediction model by multitask learning in which output characteristics of a plurality of different conditions are simultaneously learned by a neural network or a Gaussian process, or the like, for example (refer to Patent Document 1 or the like).

### PRIOR ART DOCUMENTS

### PATENT DOCUMENTS

Patent Document 1: Japanese Laid-Open Patent Publication No. 2020-95310

### DISCLOSURE OF THE INVENTION

### PROBLEM TO BE SOLVED BY THE INVENTION

However, in the conventional method for improving the prediction accuracy when the number of data is small, a sufficiently high prediction accuracy may not be obtainable when using a large number of data measured at the room temperature and a small number of data measured at the high temperature or the low temperature other than the room temperature.

One object of the present disclosure is to provide a material characteristics prediction method, a material characteristics prediction program, a material characteristics prediction device, a model generation method, a model generation program, and a model generation device, capable of accurately predicting material characteristics that vary depending on the temperature.

### MEANS OF SOLVING THE PROBLEM

The present disclosure includes the following configurations.
[1] A material characteristics prediction method for predicting material characteristics of a target material, comprising:
   model setting step of setting a trained model acquired by machine learning of a correspondence relationship between an explanatory variable including information related to a material composition or a manufacturing condition of the target material, and an objective variable including information related to the material characteristics of the target material; and
   prediction step of inputting an explanatory variable related to a target material whose material characteristics is to be predicted to the trained model set in the model setting step, and outputting an objective variable related to information of the explanatory variable, so as to predict the material characteristics of the target material to be predicted based on the objective variable,
   wherein the explanatory variable further includes information related to at least one of a material characteristics evaluation temperature that is a temperature at a time of measurement of the material characteristics included in the objective variable, and an evaluation temperature holding time that is a time during which the material characteristics evaluation temperature is held until the measurement of the material characteristics.
[2] The material characteristics prediction method according to [1], wherein the trained model is a neural network.
[3] The material characteristics prediction method according to [2], wherein
   the material characteristics predicted in the prediction step are characteristics of a metal material, a polymer material, or a glass material that vary in an S-shape depending on the material characteristics evaluation temperature or the evaluation temperature holding time, and
   an S-shaped function, including a hyperbolic tangent function or a sigmoid function, is used as an activation function of the neural network.
[4] The material characteristics prediction method according to [1], wherein a kernel method is applied as a machine learning method of the trained model.
[5] The material characteristics prediction method according to [4], wherein
   the material characteristics predicted in the prediction step are characteristics of a metal material, a polymer material, or a glass material that vary in an S-shape depending on the material characteristics evaluation temperature or the evaluation temperature holding time, and
   an inverse sine function is used as a kernel function of the kernel method.
[6] A material characteristics prediction program for predicting material characteristics of a target material, the material characteristics prediction program causing a computer to implement:
   a model setting function that sets a trained model acquired by machine learning of a correspondence relationship between an explanatory variable including information related to a material composition or a manufacturing condition of the target material, and an objective variable including information related to the material characteristics of the target material; and
   a prediction function that inputs an explanatory variable related to a target material whose material characteristics is to be predicted to the trained model set in the model setting function, and outputs an objective variable related to information of the explanatory variable, so as to predict the material characteristics of the target material to be predicted based on the objective variable,
   wherein the explanatory variable further includes information related to at least one of a material characteristics evaluation temperature that is a temperature at a time of measurement of the material characteristics included in the objective variable, and an evaluation temperature holding time that is a time during which the material characteristics evaluation temperature is held until the measurement of the material characteristics.
[7] A material characteristics prediction device for predicting material characteristics of a target material, comprising:
   a trained model acquired by machine learning of a correspondence relationship between an explanatory variable including information related to a material composition or a manufacturing condition of the target material, and an objective variable including information related to the material characteristics of the target material; and
   a predicting part configured to input an explanatory variable related to a target material whose material characteristics is to be predicted to the trained model, and output an objective variable related to information of the explanatory variable, so as to predict the material characteristics of the target material to be predicted based on the objective variable,
   wherein the explanatory variable further includes information related to at least one of a material characteristics evaluation temperature that is a temperature at a time of measurement of the material characteristics included in the objective variable, and an evaluation temperature holding time that is a time during which the material characteristics evaluation temperature is held until the measurement of the material characteristics.
[8] A model generation method for generating a model for predicting material characteristics of a target material, the model generation method comprising:
   training data creating step of acquiring a training data set including information related to a material composition or a manufacturing condition of the target material, material characteristics, and a measurement condition at a time when the material characteristics are measured; and
   model training step of generating a trained model by performing machine learning so that an input-output relationship of the model approaches an input-output relationship of the training data set, using the training data set created in the training data creating step, by regarding the information related to the material composition or the manufacturing condition and the measurement condition as an input of the model, and information related to the material characteristics as an output of the model,
   wherein the measurement condition includes at least one of a material characteristics evaluation temperature that is a temperature at a time of measurement of the material characteristics included in the output of the model, and an evaluation temperature holding time that is a time during which the material characteristics evaluation temperature is held until the measurement of the material characteristics.
[9] A model generation program for generating a model for predicting material characteristics of a target material, the model generation program causing a computer to implement:
   a training data creating function that acquires a training data set including information related to a material composition or a manufacturing condition of the target material, material characteristics, and a measurement condition at a time when the material characteristics are measured; and
   a model training function that generates a trained model by performing machine learning so that an input-output relationship of the model approaches an input-output relationship of the training data set, using the training data set created in the training data creating function, by regarding the information related to the material composition or the manufacturing condition and the measurement condition as an input of the model, and information related to the material characteristics as an output of the model,
   wherein the measurement condition includes at least one of a material characteristics evaluation temperature that is a temperature at a time of measurement of the material characteristics included in the output of the model, and an evaluation temperature holding time that is a time during which the material characteristics evaluation temperature is held until the measurement of the material characteristics.
[10] A model generation device for generating a model for predicting material characteristics of a target material, the model generation device comprising:
   a training data creating part configured to create a training data set including information related to a material composition or a manufacturing condition of the target material, material characteristics, and a measurement condition at a time when the material characteristics are measured; and
   a model training part configured to generate a trained model by performing machine learning so that an input-output relationship of the model approaches an input-output relationship of the training data set, using the training data set created in the training data creating part, by regarding the information related to the material composition or the manufacturing condition and the measurement condition as an input of the model, and information related to the material characteristics as an output of the model,
   wherein the measurement condition includes at least one of a material characteristics evaluation temperature that is a temperature at a time of measurement of the material characteristics included in the output of the model, and an evaluation temperature holding time that is a time during which the material characteristics evaluation temperature is held until the measurement of the material characteristics.

### EFFECTS OF THE INVENTION

According to the present disclosure, it is possible to provide a material characteristics prediction method, a material characteristics prediction program, a material characteristics prediction device, a model generation method, a model generation program, and a model generation device, capable of accurately predicting material characteristics that vary depending on the temperature.

### BRIEF DESCRIPTION OF DRAWINGS

[FIG. 1] FIG. 1 is a functional block diagram of a material characteristics prediction device according to one embodiment.
[FIG. 2] FIG. 2 is a functional block diagram of a model generation device according to one embodiment.
[FIG. 3] FIG. 3 is a hardware configuration diagram of the material characteristics prediction device and the model generation device.
[FIG. 4] FIG. 4 is a diagram illustrating an evaluation temperature dependency of a tensile strength of an aluminum alloy.
[FIG. 5] FIG. 5 is a diagram illustrating a holding time dependency of the tensile strength of the aluminum alloy.
[FIG. 6] FIG. 6 is a diagram illustrating the number of data for each material characteristics evaluation temperature, related to a measurement data group of the material characteristics of the aluminum alloy acquired from a public database.
[FIG. 7] FIG. 7 is a flow chart of a material characteristics prediction process according to one embodiment.
[FIG. 8] FIG. 8 is a flow chart of a model generation process according to one embodiment.
[FIG. 9] FIG. 9 is a diagram illustrating a verification data set used for learning and prediction of a prediction model.
[FIG. 10] FIG. 10 is a diagram illustrating a configuration of the prediction model used in an exemplary implementation 1.
[FIG. 11] FIG. 11 is a diagram illustrating the configuration of the prediction model used in a comparative example 1.
[FIG. 12] FIG. 12 is a diagram illustrating prediction results of the exemplary implementation 1 and the comparative example 1.
[FIG. 13] FIG. 13 is a diagram illustrating interpolation prediction results of exemplary implementations 4 and 5.
[FIG. 14] FIG. 14 is a diagram illustrating the interpolation prediction results of exemplary implementations 2 and 3.
[FIG. 15] FIG. 15 is a diagram illustrating extrapolation-prediction results of the exemplary implementations 4 and 5.
[FIG. 16] FIG. 16 is a diagram illustrating the extrapolation-prediction results of the exemplary implementations 2 and 3.
[FIG. 17] FIG. 17 is an enlarged view of a vicinity of each plotted position at each part in FIG. 15.
[FIG. 18] FIG. 18 is an enlarged view of the vicinity of each plotted position at each part in FIG. 16.

### MODE OF CARRYING OUT THE INVENTION

Hereinafter, embodiments will be described with reference to the accompanying drawings. In order to facilitate understanding of the description, the same constituent elements are designated by the same reference numerals where possible in the drawings, and a redundant description thereof will be omitted.

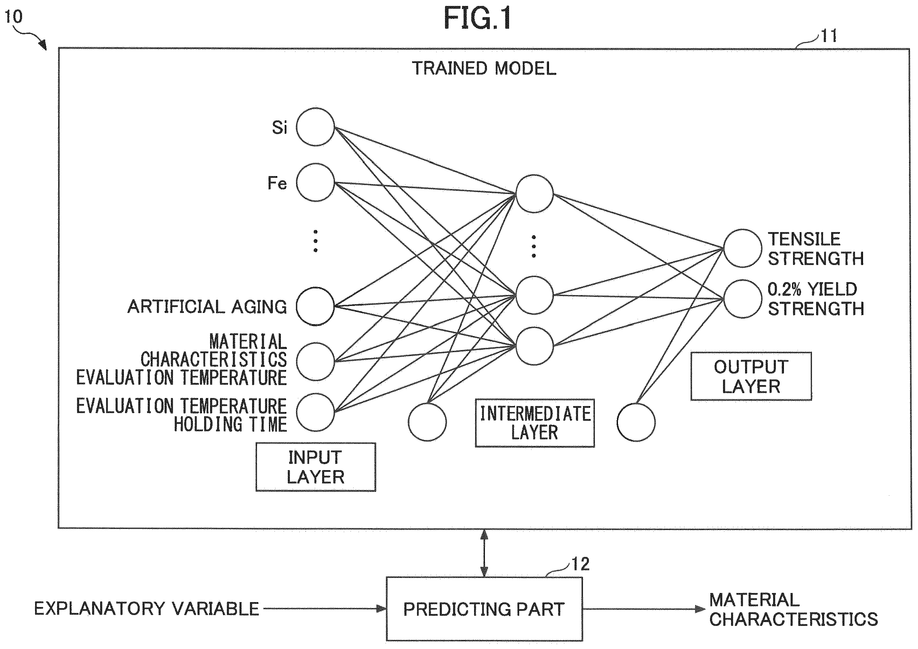

FIG. 1 is a functional block diagram of a material characteristics prediction device 10 according to one embodiment. The material characteristics prediction device 10 is a device for predicting material characteristics of a target material, including a material having a plurality of compositions or a material manufactured by a combination of a plurality of manufacturing conditions.

As illustrated in FIG. 1, the material characteristics prediction device 10 includes a trained model 11, and a predicting part 12.

The trained model 11 is a model acquired by machine learning of a correspondence relationship between an explanatory variable including information related to the material composition or the manufacturing condition of the target material, and an objective variable including information related to the material characteristics of the target material. Based on an explanatory variable, related to a target material whose material characteristics is to be predicted and input to the trained model 11, the trained model 11 outputs an objective variable related to information of the input explanatory variable. The trained model is preferably a neural network.

In the present embodiment, the trained model 11 is a three-layer neural network having an input layer, an intermediate layer, and an output layer, as illustrated in FIG. 1. The number of nodes in the input layer of the trained model 11 is the same as the number of items of the explanatory variable, and a numerical value of each item is input to the corresponding node. In FIG. 1, a numerical value corresponding to a weight percentage (wt%) of an additive element, such as Si, Fe, or the like, is input to a corresponding node of the input layer as the information related to the material composition. In addition, a numerical value (for example, 0 or 1) corresponding to whether or not an artificial aging is performed or the like, is input to a corresponding node of the input layer as the information related to the manufacturing condition. Moreover, the number of nodes in the output layer of the trained model 11 is the same number as the number of items of the objective variable, and a numerical value of each item is output from the corresponding node. In FIG. 1, the numerical values corresponding to a tensile strength, a 0.2% yield stress, or the like are output from the corresponding nodes of the output layer, as the information related to material characteristics.

In addition, as illustrated in FIG. 1, the trained model 11 may have a configuration in which a bias node is coupled to each node of the intermediate layer and each node of the output layer. Moreover, it is possible to use a deep neural network including two or more intermediate layers. However, when the number of intermediate layers is increased to two or more layers, the number of parameters, that is, the number of networks, increases, and overlearning of the training data occurs such that a prediction accuracy with respect to test data tends to decrease. For this reason, it is more preferable for the intermediate layer to be a single layer.

Although not illustrated in FIG. 1, in a case where the material characteristics of an aluminum alloy is to be predicted, the material composition of the explanatory variable may include additional elements, such as Si, Fe, Cu, Mn, Mg, Cr, Ni, Zn, Ti, V, Pb, Sn, B, Bi, Zr, Li, B, Ga, P, or the like. In addition, the manufacturing condition of the explanatory variable may include a heat treatment temperature and a heat treatment time of treatments, such as annealing, solutionizing, artificial aging, natural aging, stabilizing, high-temperature working, cold working, or the like, and a processing condition of processes, such as extrusion, forging, drawing, rolling, or the like. Moreover, the material characteristics of the objective variable may include a tensile strength, a 0.2% yield stress, an elongation, a linear expansion coefficient, a Young's modulus, a fatigue strength, a hardness, a Poisson's ratio, a creep characteristic, a shear strength, a specific heat, a thermal conductivity, an electric conductivity, a density, or the like.

In the present embodiment in particular, the explanatory variable further includes information related to a "material characteristics evaluation temperature", which is a temperature at a time of measurement of the material characteristics included in the objective variable, and an "evaluation temperature holding time", which is a time during which the material characteristics evaluation temperature is held until the measurement of the material characteristics. That is, the input information of the trained model 11 includes the information related to the material characteristics evaluation temperature and the evaluation temperature holding time, and numerical values corresponding to the material characteristics evaluation temperature and the evaluation temperature holding time are input to corresponding nodes of the input layer.

The explanatory variable need only include at least one of the "material characteristics evaluation temperature" and the "evaluation temperature holding time", and only the "material characteristics evaluation temperature" may be included in the explanatory variable, or only the "evaluation temperature holding time" may be included in the explanatory variable.

The predicting part 12 inputs the explanatory variable related to the target material whose material characteristics are to be predicted, to the trained model 11, outputs the objective variable related to the input explanatory variable, and predicts the material characteristics of the target material to be predicted, based on the output objective variable.

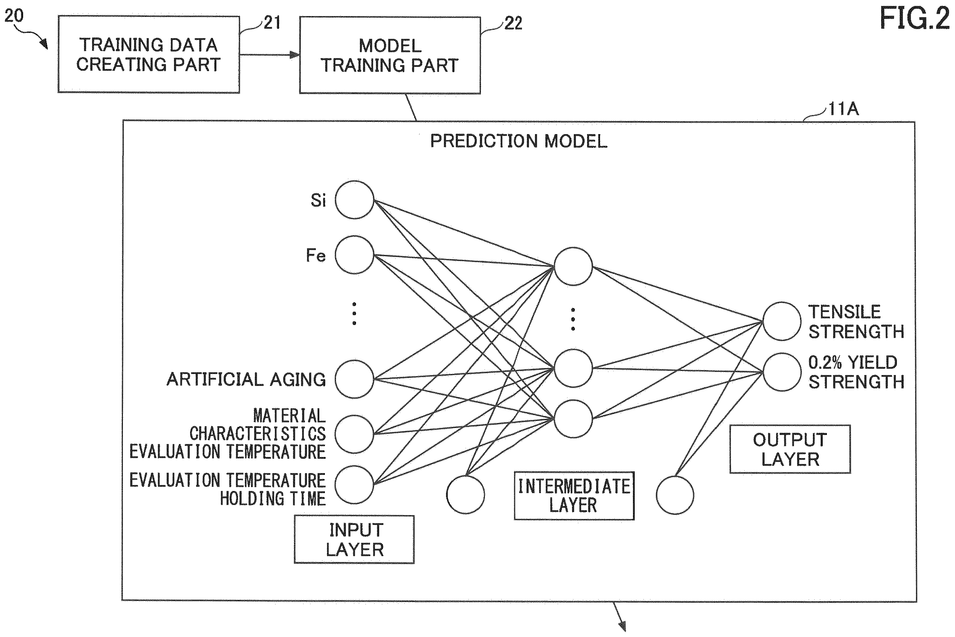

The trained model 11 used in the material characteristics prediction device 10 can be generated by the model generation device 20 illustrated in FIG. 2. FIG. 2 is a functional block diagram of the model generation device 20 according to one embodiment.

As illustrated in FIG. 2, the model generation device 20 includes a training data creating part 21, a model training part 22, and a prediction model 11A. The prediction model 11A is a model before completion of the machine learning of the trained model 11 illustrated in FIG. 1, and is used for predicting the material characteristics of the target material. A configuration of the prediction model 11A is the same as that of the trained model 11 described with reference to FIG. 1.

The training data creating part 21 creates a training data set, including the information related to the material composition or the manufacturing condition of the target material, the material characteristics, and a measurement condition at the time of measurement of the material characteristics.

The model training part 22 trains the prediction model 11A using the training data set created by the training data creating part 21, to generate a trained model. The model training part 22 generates the trained model 11 by performing machine learning so that an input-output relationship of the prediction model 11A approaches an input-output relationship of the training data set, by regarding the information related to the material composition or the manufacturing condition and the measurement condition as the input of the prediction model 11A, and information related to the material characteristics as the output of the prediction model 11A.

In a case where the prediction model 11A is a neural network, the model training part 22 can train the prediction model 11A using a known machine learning method, such as a backpropagation method, a batch gradient descent method, a stochastic gradient descent method, a mini-batch gradient descent method, a variational Bayesian method, or the like.

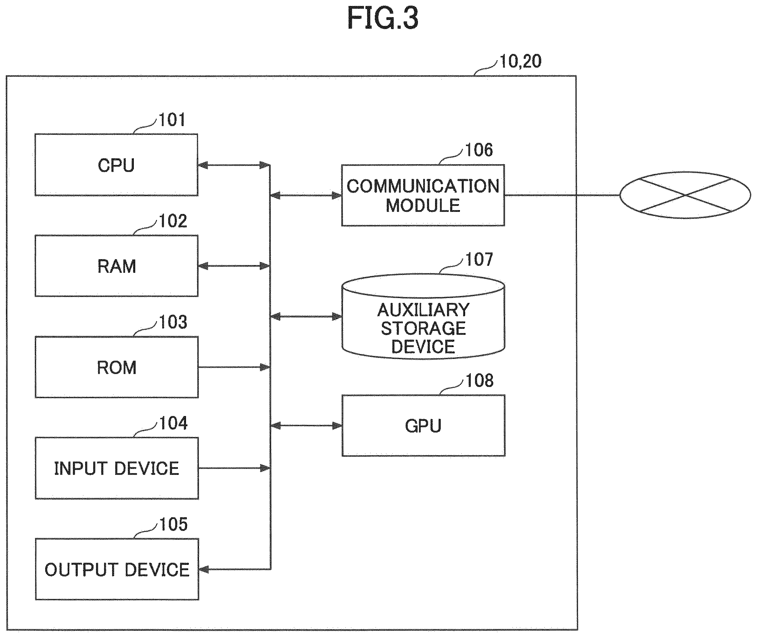

FIG. 3 is a hardware configuration diagram of the material characteristics prediction device 10 and the model generation device 20. As illustrated in FIG. 3, the material characteristics prediction device 10 and the model generation device 20 can be physically configured by a computer system including, among other things, a central processing unit (CPU) 101, a graphics processing unit (GPU) 108, a random access memory (RAM) 102 and a read only memory (ROM) 103 as main storage devices, an input device 104 such as a keyboard, a mouse, or the like, an output device 105, such as a display or the like, a communication module 106 that is a data transmitting and receiving device, such as a network card or the like, an auxiliary storage device 107, such as a hard disk or the like.

Each function of the material characteristics prediction device 10 illustrated in FIG. 1 can be implemented by reading predetermined computer software (a material characteristics prediction program) into hardware including the CPU 101, the RAM 102, or the like, operating the communication module 106, the input device 104, and the output device 105 under a control of the CPU 101, and reading and writing with respect to the RAM 102 and the auxiliary storage device 107. That is, by causing a computer to execute the material characteristics prediction program according to the present embodiment, the material characteristics prediction device 10 functions as the trained model 11 and the predicting part 12 illustrated in FIG. 1.

Similarly, each function of the model generation device 20 illustrated in FIG. 2 can be implemented by reading predetermined computer software (a model generation program) into hardware including the CPU 101, the RAM 102, or the like, operating the communication module 106, the input device 104, and the output device 105 under the control of the CPU 101, and reading and writing with respect to the RAM 102 and the auxiliary storage device 107. That is, by causing the computer to execute the model generation program according to the present embodiment, the model generation device 20 functions as the training data creating part 21, the model training part 22, and the prediction model 11A illustrated in FIG. 2.

The material characteristics prediction program and the model generation program according to the present embodiment are stored in a storage device of the computer, for example. A part or all of the material characteristics prediction program and the model generation program may be transmitted via a transmission medium, such as a communication line or the like, received by the communication module 106 or the like of the computer, and recorded (including being installed). In addition, the material characteristics prediction program and the model generation program may be configured to be recorded (including being installed) in the computer from a state where a part or all of the material characteristics prediction program and the model generation program is stored in a portable storage medium, such as a CD-ROM, a DVD-ROM, a flash memory, or the like.

Similarly, the trained model 11 generated by the model generation device 20 may be configured to be stored in the portable storage medium that is portable by itself, transmitted via the transmission medium, or recorded in the computer.

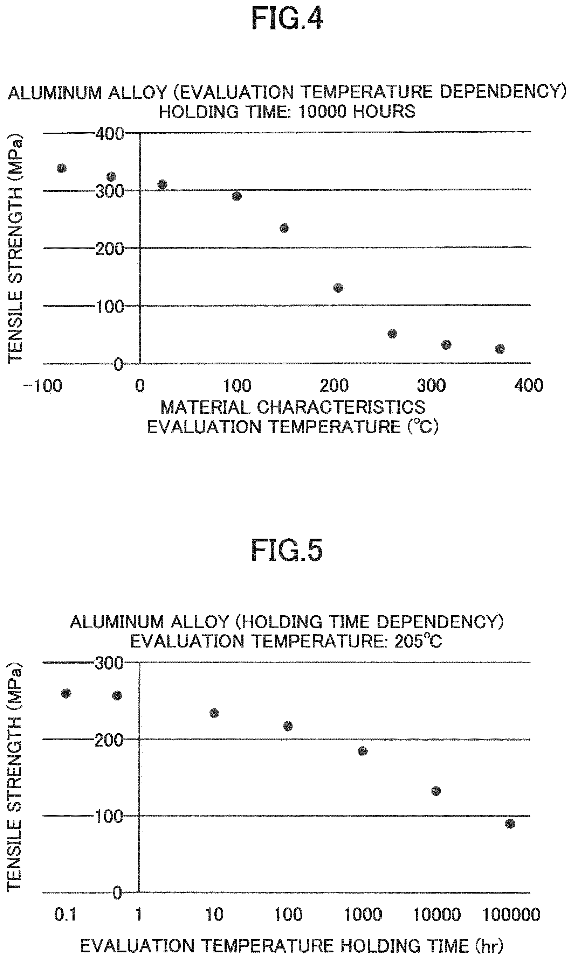

In the present embodiment, the activation function used for the node of the intermediate layer of the trained model 11 preferably has a characteristic common to an evaluation temperature dependency and a holding time dependency of the material characteristics of the objective function. In addition, the aluminum alloy is described as an example of the target material, and the tensile strength is described as an example of the material characteristics of the objective function. FIG. 4 is a diagram illustrating the evaluation temperature dependency of the tensile strength of the aluminum alloy. FIG. 5 is a diagram illustrating the holding time dependency of the tensile strength of the aluminum alloy.

In FIG. 4, the abscissa indicates the material characteristics evaluation temperature (°C), and the ordinate indicates the tensile strength (MPa). In FIG. 4, values of the tensile strength measured after the same holding time (10000 hours) at a plurality of material characteristics evaluation temperatures are plotted, and a characteristic (evaluation temperature dependency) in which the tensile strength varies depending on the material characteristics evaluation temperature is illustrated. As illustrated in FIG. 4, the tensile strength has a tendency to decrease more toward the high-temperature side in the entire temperature range, and a rate of change of this decreasing tendency is relatively large and sharply decreases in a steep slope within a range higher than or equal to approximately 100°C and lower than or equal to approximately 250°C. A curve of this decreasing tendency has an inflection point at a position of approximately 200°C. In other words, as illustrated in FIG. 4, the tensile strength has a characteristic varying in an S-shape depending on the material characteristics evaluation temperature.

In FIG. 5, the abscissa indicates the evaluation temperature holding time (hr (hours)), and the ordinate indicates the tensile strength (MPa). The abscissa in FIG. 5 is indicated on a logarithmic scale. In FIG. 5, values of the tensile strength measured after a plurality of evaluation temperature holding times at the same evaluation temperature (205°C) are plotted, and a characteristic (holding time dependency) in which the tensile strength varies depending on the evaluation temperature holding time is illustrated. As illustrated in FIG. 5, the tensile strength has a tendency to generally decrease toward the longer-time side, and a curve of this decreasing tendency has an inflection point at positions between 1000 hours and 10000 hours. That is, as illustrated in FIG. 5, the tensile strength has a characteristic varying in an S-shape depending on the evaluation temperature holding time.

As described above, in a case where the material characteristics of the target material predicted by the predicting part 12 is the characteristic varying in the S-shape depending on the material characteristics evaluation temperature or the evaluation temperature holding time, it is preferable to use an S-shaped function including the hyperbolic tangent function or the sigmoid function, as the activation function of the neural network of the trained model 11.

A linear activation function (identity function) may be used as the activation function of the intermediate layer of the neural network. In addition, a linear activation function (identity function) is preferably used as the activation function of the output layer.

Next, effects of the present embodiment will be described. As in the tensile strengths illustrated in FIG. 4 and FIG. 5, the material characteristics are stable and have approximately constant values at room temperature (approximately 15°C to 40°C), but has a tendency to vary greatly as the evaluation temperature shifts to the high-temperature side or the evaluation temperature holding time shifts to a longer-time side. In recent years, due to increased needs for weight reduction in automobiles or the like, there are demands to develop aluminum alloys or the like that can exhibit high strength even at high temperatures of 150°C or higher, for example, and it is desirable to be able to accurately predict the material characteristics even under a high-temperature condition in which the material characteristics greatly vary.

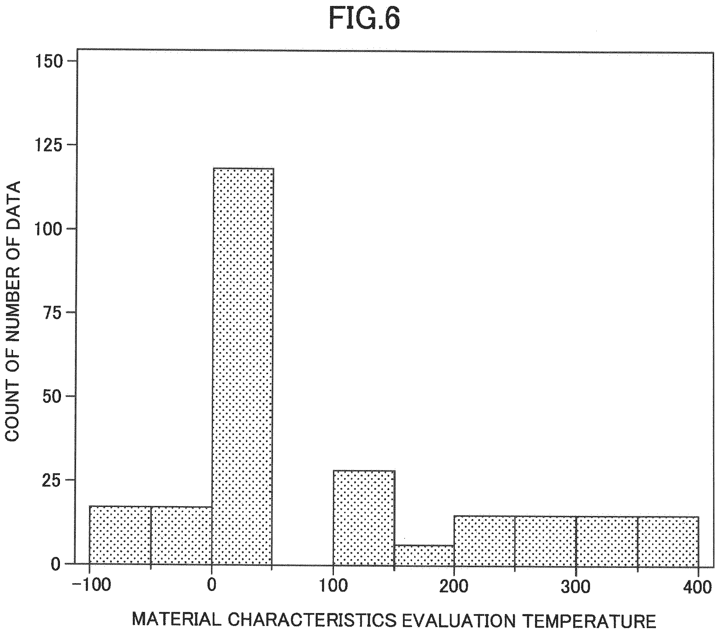

However, the prediction accuracy of the material characteristics by machine learning greatly depends on the number of measurement data. In general, the number of measurement data of the material characteristics at the high temperature, requiring time and effort for the measurement, is smaller than the number of measurement data of the material characteristics at the room temperature. FIG. 6 illustrates an example thereof.

FIG. 6 is a diagram illustrating the number of data for each material characteristics evaluation temperature, related to a measurement data group of the material characteristics of the aluminum alloy acquired from a public database. In FIG. 6, the number of data included in each temperature range is illustrated by a bar graph for a case where the evaluation temperature is segmented for every 50°C into the temperature ranges with respect to 246 data of the 6000 series aluminum alloy having the material characteristics evaluation temperature in a range from -80°C to 371°C. Material data collected from a public database, including the US MatWeb database (http://www.matweb.com/index.aspx), and the Japan Aluminum Association database

(http://metal.matdb.jp/JAA-DB/), were used for the data group illustrated in FIG. 6. In the example illustrated in FIG. 6, it can be seen that the number of data in the temperature range including the room temperature of 0 to 50°C is extremely large, while the number of data in both the high-temperature side and the low-temperature side of the room temperature is small, as described above.

When there is a difference in the number of data that can be acquired depending on the temperature range of the material characteristics evaluation temperature as described above, that is, when there is a difference in the number of data that can be used as the training data for machine learning, it is difficult to obtain a highly accurate prediction model over the entire range of the material characteristics evaluation temperature according to the conventional machine learning method, and it is particularly difficult to increase the prediction accuracy of the characteristic prediction model on the high-temperature side or the low-temperature side.

Accordingly, in the present embodiment, as illustrated in FIG. 1, information related to the material characteristics evaluation temperature and the evaluation temperature holding time, with respect to the predicted material characteristics, is added as the explanatory variable that is the input information of the trained model 11.

In the configuration of the present embodiment in which the objective characteristic is the material characteristics, the material characteristics evaluation temperature and the evaluation temperature holding time may be regarded as information that would originally be included in the output of the model. However, in the present embodiment, such information on the material characteristics evaluation temperature and the evaluation temperature holding time is intentionally used as the input information of the model. Accordingly, it is possible to cause the model to acquire the correspondence relationship between the material characteristics evaluation temperature and the evaluation temperature holding time in the training data, and the material characteristics, thereby enabling an accurate prediction of the material characteristics corresponding to the material characteristics evaluation temperature and the evaluation temperature holding time of the trained model 11. As a result, the material characteristics prediction device 10 according to the present embodiment can accurately predict the material characteristics that vary depending on the temperature.

When the prediction accuracy of the material characteristics can be improved as described above, the number of trials for designing the material having the desired characteristics can be reduced. In addition, by using a generalization ability of the prediction model, it is possible to predict the material characteristics even under design conditions not described in the database.

Moreover, in the present embodiment, when the trained model 11 is a neural network, and the material characteristics of the target material predicted by the predicting part 12 exhibit a characteristic that varies in an S-shape depending on the material characteristics evaluation temperature or the evaluation temperature holding time, an S-shaped function, including a hyperbolic tangent function or a sigmoid function, is used as the activation function of the neural network of the trained model 11.

According to this configuration, because the activation function has a characteristic that is common to the evaluation temperature dependency and the holding time dependency of the material characteristics of the objective function, a function approximation capability of the trained model 11 can be improved, and the material characteristics that vary depending on temperature can be predicted with an even higher accuracy. As illustrated in FIG. 4, it is known that a metal material generally exhibits a behavior in which the strength rapidly decreases at a certain temperature. For this reason, it may be regarded that the use of the S-shaped activation function, such as a hyperbolic tangent function, a sigmoid function, an error function, an arctangent function, or the like, that reproduces this behavior, contributes to the improvement of prediction accuracy at the high temperature. Accordingly, the effect of improving the prediction accuracy by using the S-shaped function as the activation function is particularly notable in the prediction of the material characteristics at the high temperature at which the number of measurement data is generally small or no measurement data exists.

The configuration in which the S-shaped function is used as the activation function can also be applied to predicting characteristics of a polymer material or a glass material whose characteristics rapidly vary at a glass transition temperature, and the effect of improving the prediction accuracy can be achieved similar to the present embodiment. That is, the target material whose material characteristics are to be predicted in the present embodiment may be a polymer material or a glass material, other than a metal.

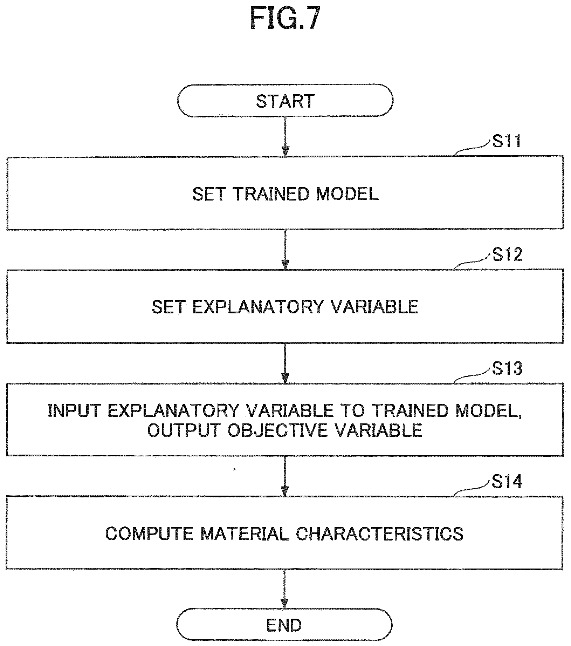

FIG. 7 is a flow chart of material characteristics prediction process according to one embodiment. Each process of the flow chart illustrated in FIG. 7 is performed by the material characteristics prediction device 10.

In step S11 (model setting step), the trained model 11 is set. A model generated in advance by the model generation device 20, or a model acquired by other methods, may be used for the trained model 11.

In step S12, an explanatory variable used for prediction is set.

In step S13 (prediction step), the predicting part 12 inputs the explanatory variable set in step S12 to the trained model 11 set in step S11, and outputs an objective variable corresponding to the explanatory variable.

In step S14, the predicting part 12 computes the material characteristics corresponding to the explanatory variable set in step S12 from the objective variable output in step S13.

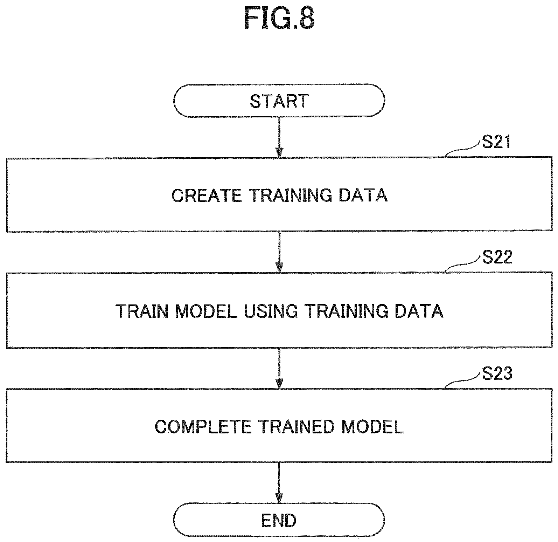

FIG. 8 is a flow chart of a model generation process according to one embodiment. Each process of the flow chart illustrated in FIG. 8 is performed by the model generation device 20.

In step S21 (training data creating step), the training data creating part 21 generates the training data used for the model training. The training data may be created by acquiring desired information from the existing public database illustrated in FIG. 6, for example, or may be created by using the actually measured data.

In step S22 (model training step), the model training part 22 trains the prediction model 11A using the training data created in step S21.

In step S23, the training of the prediction model 11A by the model training part 22 is completed, and the trained model 11 is completed.

Although the neural network is illustrated as an example of the model for predicting the material characteristics in the present embodiment, a model other than the neural network may be used as long as the model can acquire the input-output relationship between the explanatory variable and the objective variable by the machine learning. A high-temperature material characteristics may be predicted by a Gaussian process, for example. In this case, it is preferable to use an inverse sine function as the kernel function. As the machine learning method to which the kernel method is applied, a kernel ridge, a support vector machine, or the like, other than the Gaussian process, may be used.

### [Exemplary Implementations]

Next, exemplary implementations of the present invention will be specifically described.

### <Effects of Model Input Information>

An exemplary implementation 1 and a comparative example 1 were set as described below, and effects of the presence or absence of the material characteristics evaluation temperature and the evaluation temperature holding time added to the input information of the prediction model, on the prediction accuracy of the material characteristics, was verified.

### [Exemplary Implementation 1]

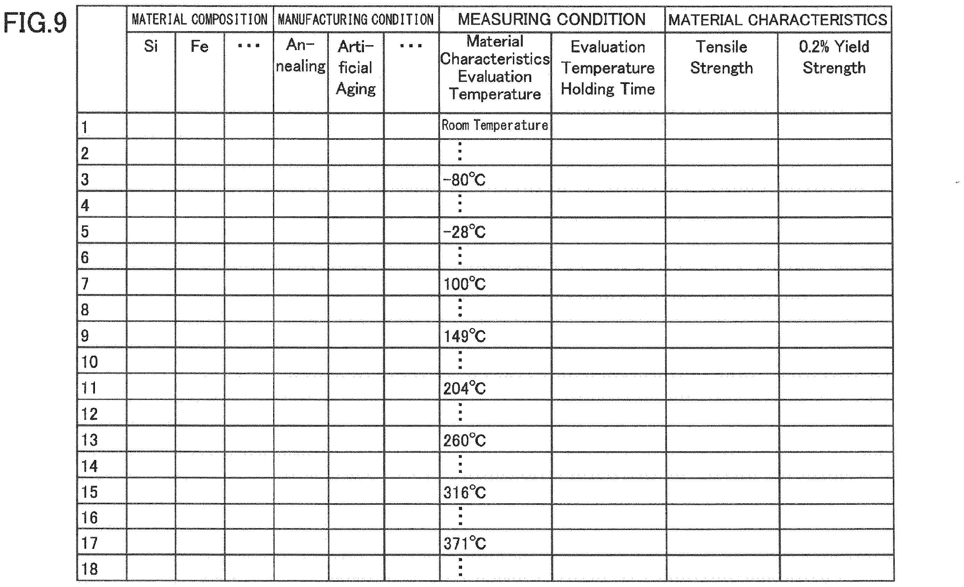

FIG. 9 is a diagram illustrating a verification data set used for learning and prediction of the prediction model. As illustrated in FIG. 9, 246 verification data sets were created using the data acquired from the public database illustrated in FIG. 6, so that each verification data set is associated with a material composition (Si, Fe, or the like), a manufacturing condition (annealing, artificial aging, or the like), a measurement condition (material characteristics evaluation temperature and evaluation temperature holding time), and material characteristics (tensile strength, 0.2% yield stress). As illustrated in FIG. 9, the verification data set includes data of nine types of material characteristics evaluation temperature, including the room temperature, -80°C, - 28°C, 100°C, 149°C, 204°C, 260°C, 316°C, and 371°C.

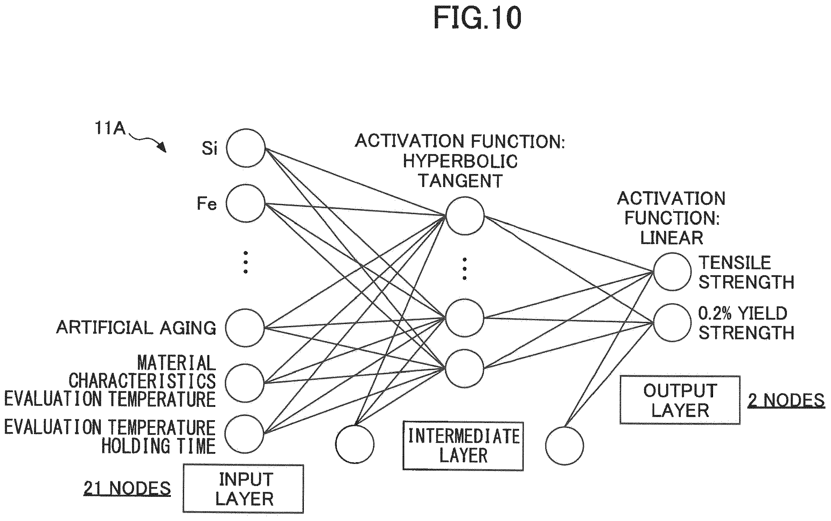

FIG. 10 is a diagram illustrating a configuration of the prediction model 11A used in the exemplary implementation 1. As illustrated in FIG. 10, the prediction model 11A according to the present embodiment described with reference to FIG. 2, that is, a model in which the material characteristics evaluation temperature and the evaluation temperature holding time are included in the input information of the three-layer neural network, was created. The explanatory variable input to the input layer of the model includes the "material composition", the "manufacturing condition", and the "measurement condition" of the verification data set illustrated in FIG. 9. The number of nodes in the input layer of the prediction model 11A is 21. The objective variable output from the output layer of the model includes two "material characteristics" (tensile strength and 0.2% yield stress) of the verification data set illustrated in FIG. 9. The number of nodes in the output layer of the prediction model 11A is two. A hyperbolic tangent function was applied to the activation function of the intermediate layer of the prediction model 11A, and a linear function was applied to the activation function of the output layer.

The material characteristics evaluation temperature of the prediction model 11A illustrated in FIG. 10 was predicted using the verification data set illustrated in FIG. 9. In the data of a category 1 of 63 sets among the 246 verification data sets, the material characteristics were measured at all of the nine types of material characteristics evaluation temperatures including the room temperature, -80°C, -28°C, 100°C, 149°C, 204°C, 260°C, 316°C, and 371°C for an evaluation temperature holding time of 10000 hours. In the data of a category 2 of the remaining 183 sets, the material characteristics evaluation temperature may be other than the nine types, the material characteristics may be measured only some of the nine types of the material characteristics evaluation temperature, or the evaluation temperature holding time may be other than 10000 hours. Among such sets, 27 sets of the data of the category 1 are used as the prediction data, and a total of 219 sets of data, including the data of the category 1 (the remaining 36 sets) and the data of the category 2 (183 sets), are used as the training data. Then, training of the prediction model 11A was performed using the training data, and the prediction using the trained model 11 after completion of training was performed using the "material composition", the "manufacturing condition", and the "measurement condition" of the prediction data. The batch gradient descent method (L-BFGS method) implemented in a scikit-learn library of Python was used for the training of the prediction model 11A. The material characteristics (predicted value) predicted by the trained model 11 was compared with the value (measured value) in the "material characteristics" column of the prediction data, to verify the prediction accuracy. The prediction accuracy was evaluated using two indexes, namely, a root mean square error and a mean absolute error.

### [Comparative Example 1]

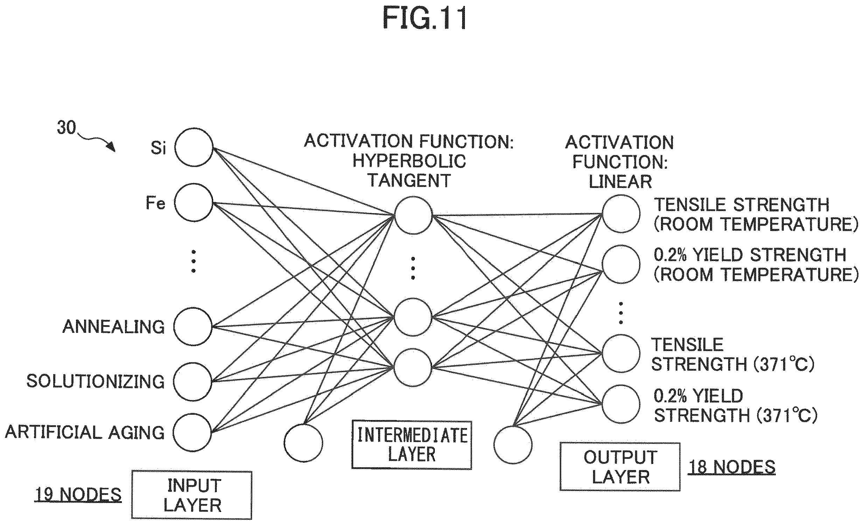

FIG. 11 is a diagram illustrating a configuration of a prediction model 30 used in a comparative example 1. As illustrated in FIG. 11, a model in which the material characteristics evaluation temperature and the evaluation temperature holding time are excluded from the prediction model 11A according to the present embodiment described with reference to FIG. 2, that is, the input information of the three-layer neural network, was created. That is, the explanatory variable input to the input layer of the prediction model 30 includes only the "material composition" and the "manufacturing condition" of the verification data set illustrated in FIG. 9, and do not include the "measurement condition". That is, the number of nodes in the input layer of the prediction model 30 is 19. The objective variable output from the output layer of the prediction model 30 includes the two "material characteristics" (tensile strength and 0.2% yield stress) of the verification data set illustrated in FIG. 9 for each of the nine types of the material characteristics evaluation temperature. That is, the number of nodes in the output layer of the prediction model 30 is 2 × 9 = 18 nodes. A hyperbolic tangent function was applied to the activation function of the intermediate layer, and a linear function was applied to the activation function of the output layer. In the configuration of the neural network of comparative example 1, only the data of the category 1 can be used for the learning and the prediction, because the data can be used for the learning and the prediction only when all the values (nine sets) of the output node are present with respect to one set of the input values. In this example, the 27 sets of the data of the category 1, which are the same as those used in the exemplary implementation 1, are used as the prediction data, and the remaining 36 sets of the data of the category 1 are used as the training data.

Other conditions were the same as those of the exemplary implementation 1, and the material characteristics evaluation temperature of the prediction model 30 illustrated in FIG. 11 was predicted using the verification data set illustrated in FIG. 9. When verifying the prediction accuracy, a reference was made to the "material characteristics evaluation temperature" of the prediction data, and the output value (predicted value) of the two nodes corresponding to the corresponding material characteristics evaluation temperature among the 18 outputs of the prediction model 30 was compared with the value (measured value) in the "material characteristics" column of the prediction data.

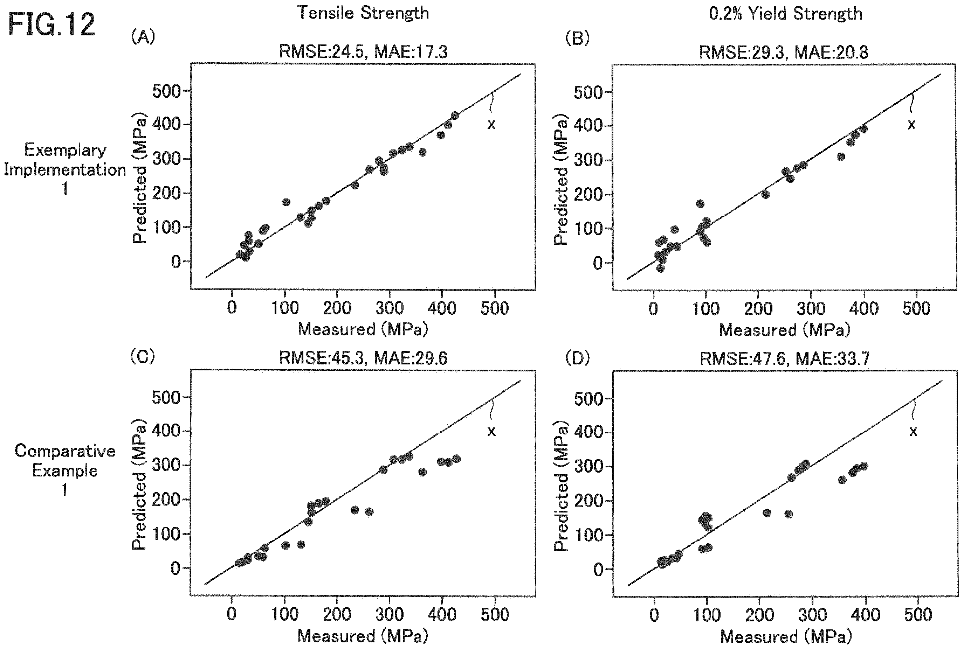

FIG. 12 is a diagram illustrating prediction results of the exemplary implementation 1 and the comparative example 1. FIG. 12(A) and FIG. 12(B) illustrate the prediction results of the tensile strength and the 0.2% yield stress, respectively, in exemplary implementation 1. FIG. 12(C) and FIG. 12(D) illustrate the prediction results of the tensile strength and the 0.2% yield stress, respectively, in the comparative example 1. In each of FIG. 12(A) through FIG. 12(D), the abscissa indicates the value (measured value) in the "material characteristic" column of the prediction data, and the ordinate indicates the material characteristics (predicted value) predicted by the trained model. In each of FIG. 12(A) through FIG. 12(D), a predicted value corresponding to a measured value is plotted for each prediction data. A straight line x illustrated in each of these figures indicates the characteristics for a case where the measured value and the predicted value are the same, and the prediction accuracy become higher as the plot approaches closer to the straight line x.

When FIG. 12(A) and FIG. 12(C) are compared, it can be seen that regarding the tensile strength among the material characteristics, a variation in the plots is smaller in the exemplary implementation 1 than in the comparative example 1, and the tensile strength is concentrated at positions close to the straight line x. The root mean square error (RMSE) was 45.3 in the comparative example 1, whereas the RMSE was 24.5 and small in the exemplary implementation 1. The mean absolute error (MAE) was 29.6 in the comparative example 1, whereas the MAE was 17.3 and small in the exemplary implementation 1.

Similarly, when FIG. 12(B) and FIG. 12(D) are compared, it can be seen that the 0.2% yield stress among the material characteristics also has a smaller variation in the plots in the exemplary implementation 1 than in the comparative example 1, and the 0.2% yield stress is concentrated at positions close to the straight line x. The root mean square error (RMSE) was 47.6 in the comparative example 1, whereas RMSE was 29.3 and small in the exemplary implementation 1. The mean absolute error (MAE) was 33.7 in the comparative example 1, whereas the MAE was 20.8 and small in the exemplary implementation 1.

The results illustrated in FIG. 12 indicate that the prediction accuracy of the material characteristics is improved by adding the material characteristics evaluation temperature and the evaluation temperature holding time to the input information (explanatory variable) of the prediction model.

### <Effects of Activation Function>

The exemplary implementations 2 and 3 and the exemplary implementations 4 and 5 were set as described below, and the effects of a variation in the activation function of the prediction model on the prediction accuracy of the material characteristics were verified.

### [Exemplary Implementation 2]

The verification data set illustrated in FIG. 9 was used to perform interpolation prediction with respect to the material characteristics evaluation temperature of the prediction model 11A illustrated in FIG. 10. More particularly, the 246 verification data sets were segmented at random into 75% (185 sets) of the training data and 25% (61 sets) of the prediction data. A hyperbolic tangent function was applied to the activation function.

Further, the verification data set illustrated in FIG. 9 was used to perform extrapolation prediction with respect the material characteristics evaluation temperature of the prediction model 11A illustrated in FIG. 10. More particularly, among the 246 verification data sets, 185 sets (75%) of data in which the material characteristics evaluation temperature becomes 150°C or lower were used as the training data, and 61 sets (25%) of data in which the material characteristics evaluation temperature becomes higher than 150°C were used as the prediction data. In the present specification, randomly segmenting the data into the training data and the prediction data is referred to as "interpolation prediction", and regarding data having a temperature of 150°C or lower as the training data and data having a temperature higher than 150°C as the prediction data is referred to as "extrapolation prediction".

### [Exemplary Implementation 3]

The interpolation prediction and the extrapolation prediction were performed similar to the exemplary implementation 2, except that a sigmoid function was applied to the activation function.

### [Exemplary Implementation 4]

The interpolation prediction and the extrapolation prediction were performed similar to the exemplary implementation 2, except that a linear function was applied to the activation function.

### [Exemplary Implementation 5]

The interpolation prediction and the extrapolation prediction were performed similar to the exemplary implementation 2, except that a normalized linear unit function was applied to the activation function.

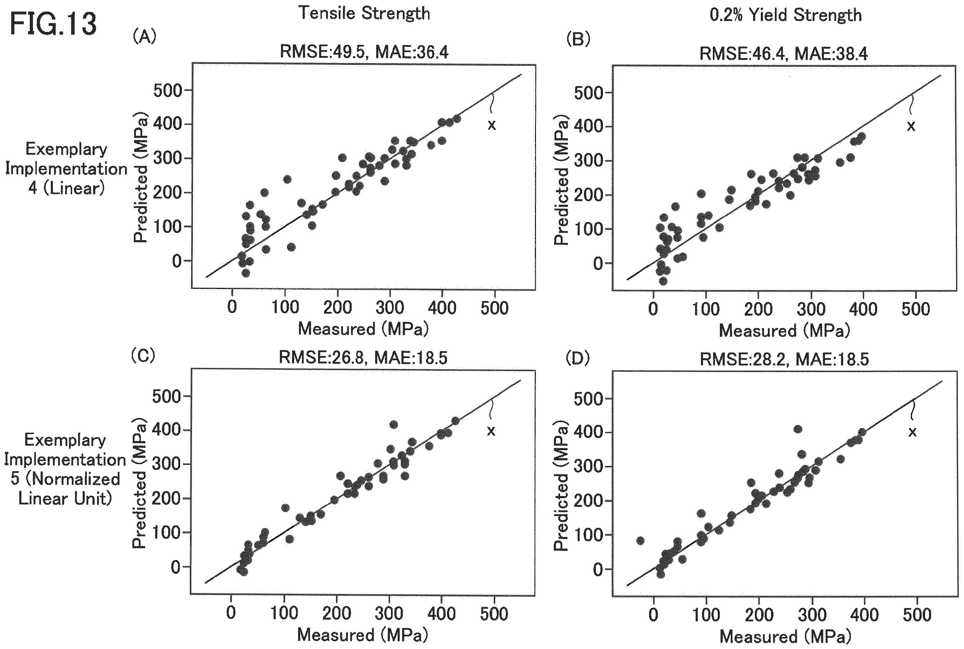

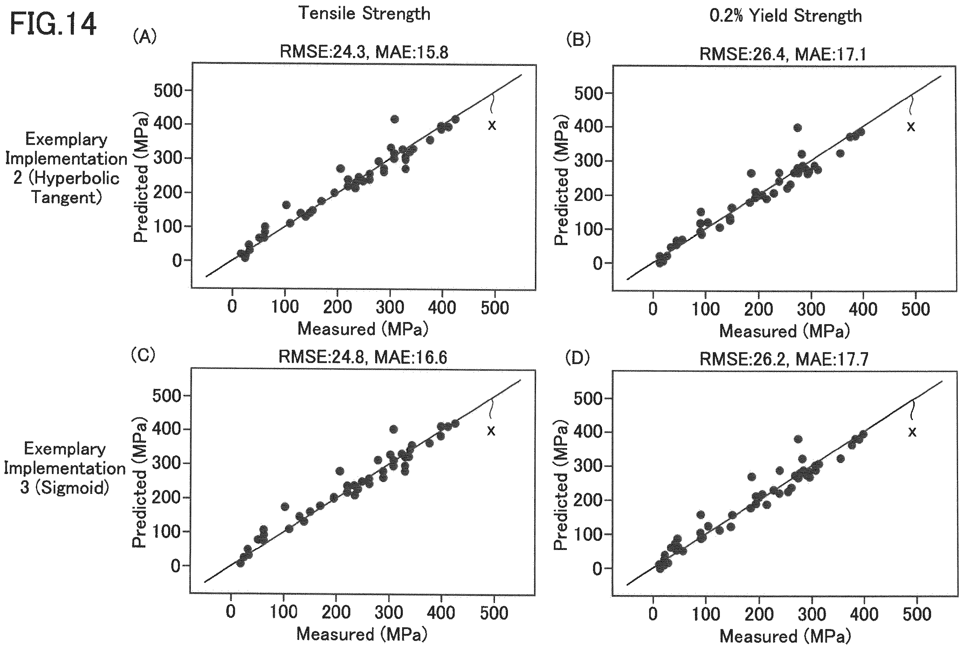

FIG. 13 is a diagram illustrating interpolation prediction results of exemplary implementations 4 and 5. FIG. 14 is a diagram illustrating interpolation prediction results of the exemplary implementations 2 and 3. The outline of FIG. 13 and FIG. 14 is the same as that of FIG. 12. From FIG. 13 and FIG. 14, regarding the tensile strength among the material characteristics, the root mean square error (RMSE) was 49.5 in exemplary implementation 4, 26.8 in the exemplary implementation 5, 24.3 in exemplary implementation 2, and 24.8 in the exemplary implementation 3. The mean absolute error (MAE) was 36.4 in the exemplary implementation 4, 18.5 in the exemplary implementation 5, 15.8 in the exemplary implementation 2, and 16.6 in the exemplary implementation 3.

In addition, regarding the 0.2% yield stress among the material characteristics, the root mean square error (RMSE) was 46.4 in the exemplary implementation 4, 28.2 in the exemplary implementation 5, 26.4 in the exemplary implementation 2, and 26.2 in the exemplary implementation 3. The mean absolute error (MAE) was 38.4 in the exemplary implementation 4, 18.5 in the exemplary implementation 5, 17.1 in the exemplary implementation 2, and 17.7 in the exemplary implementation 3.

From the results illustrated in FIG. 13 and FIG. 14, it can be seen that, with respect to the interpolation prediction, the variation in the plots is larger in the exemplary implementation 4 than in the other exemplary implementation for both the tensile strength and the 0.2% yield stress, the plots are away from the straight line x, and the prediction accuracy is relatively poor.

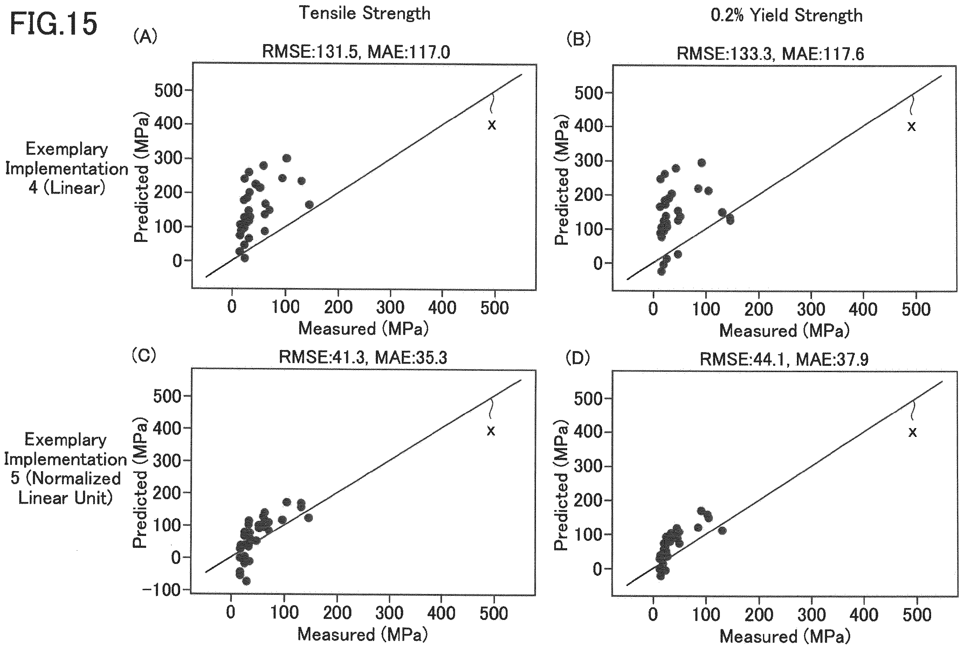

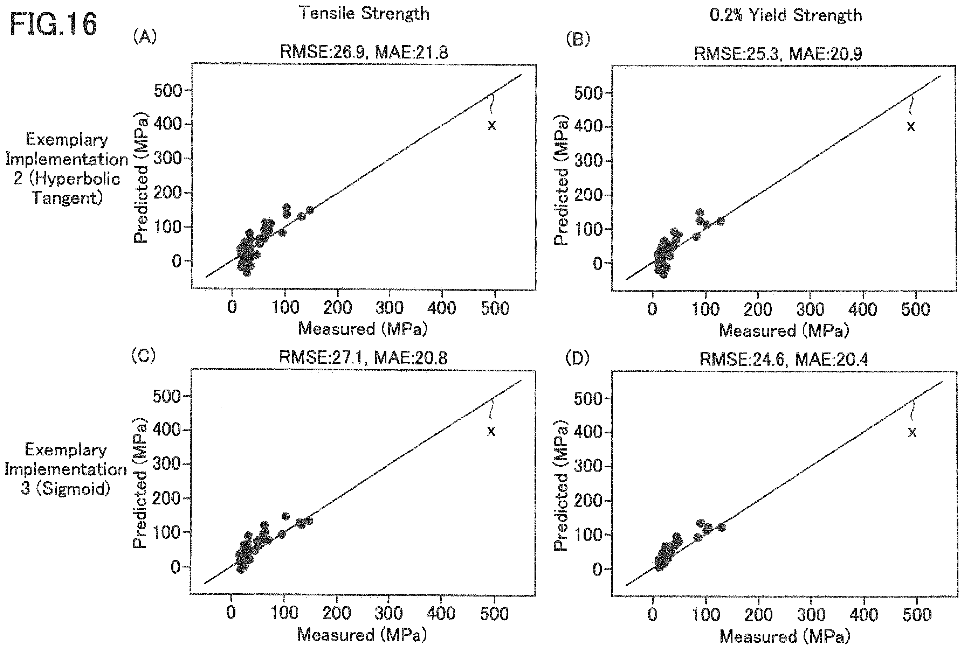

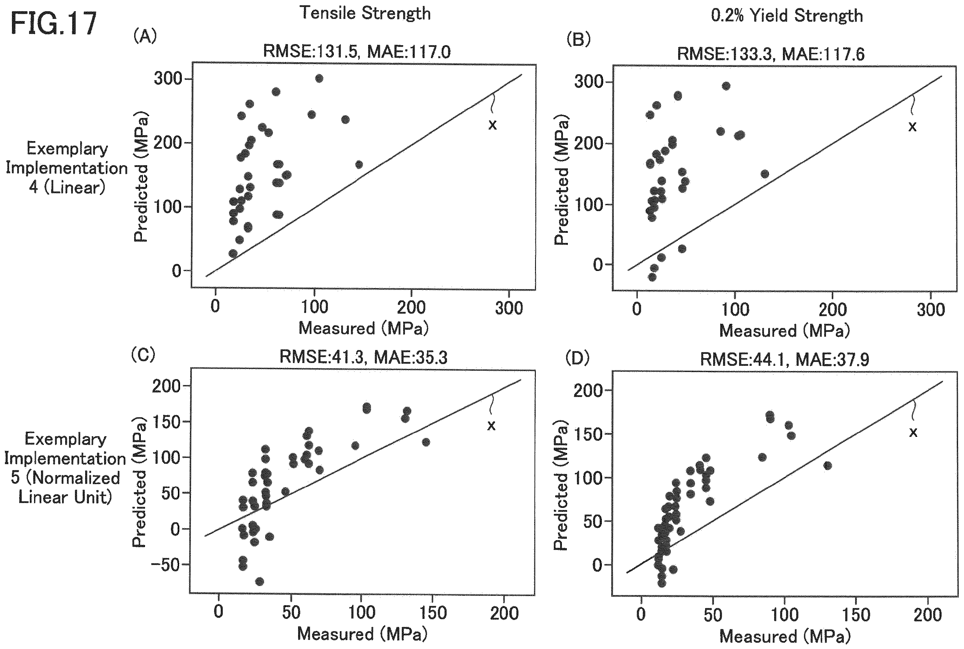

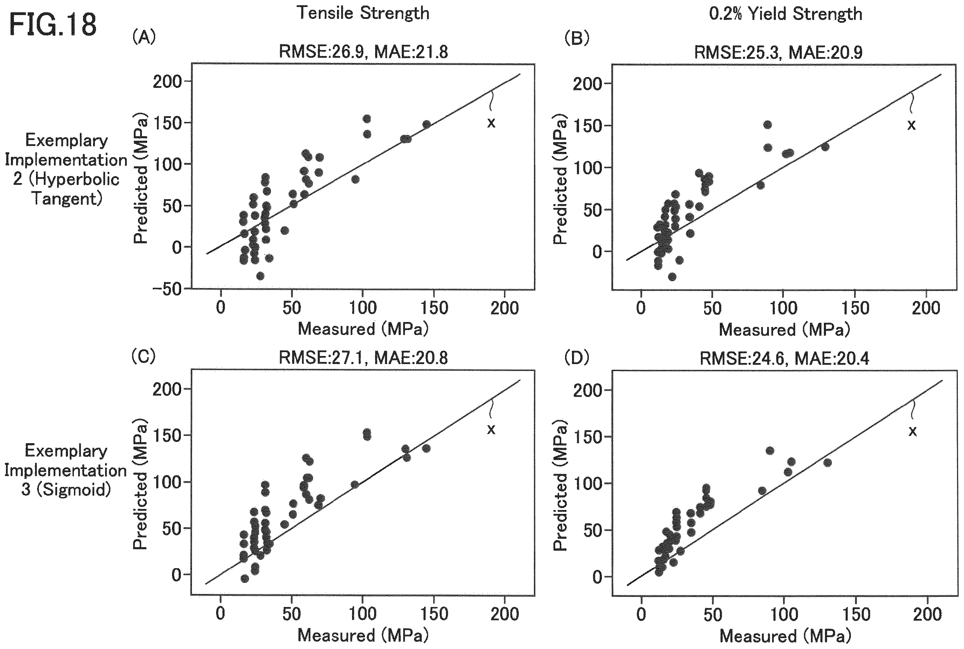

FIG. 15 is a diagram illustrating extrapolation prediction results of the exemplary implementations 4 and 5. FIG. 16 is a diagram illustrating extrapolation prediction results of the exemplary implementations 2 and 3. FIG. 17 is an enlarged view of a vicinity of each plotted position at each part in FIG. 15. FIG. 18 is an enlarged view of the vicinity of the plotted position at each part in FIG. 16. The outline of FIG. 15 through FIG. 18 is the same as that of FIG. 12.

From FIG. 15 through FIG. 18, regarding the tensile strength among the material characteristics, the root mean square error (RMSE) was 131.5 in the exemplary implementation 4, 41.3 in the exemplary implementation 5, 26.9 in the exemplary implementation 2, and 27.1 in the exemplary implementation 3. The mean absolute error (MAE) was 117.0 in the exemplary implementation 4, 35.3 in the exemplary implementation 5, 21.8 in the exemplary implementation 2, and 20.8 in the exemplary implementation 3.

Regarding the 0.2% yield stress among the material characteristics, the root mean square error (RMSE) was 133.3 in the exemplary implementation 4, 44.1 in the exemplary implementation 5, 25.3 in the exemplary implementation 2, and 24.6 in the exemplary implementation 3. The mean absolute error (MAE) was 117.6 in the exemplary implementation 4, 37.9 in the exemplary implementation 5, 20.9 in the exemplary implementation 2, and 20.4 in the exemplary implementation 3.

From the results illustrated in FIG. 15 through FIG. 18, it can be seen that, with respect to the extrapolation prediction, the variation in the plots in the exemplary implementations 2 and 3 is smaller than that in the exemplary implementations 4 and 5, for both of the tensile strength and the 0.2% yield stress, the plots are close to the straight line x, and the prediction accuracy is excellent. Accordingly, considering the results of both the interpolation prediction and the extrapolation prediction, the prediction accuracy is relatively high in the exemplary implementations 2 and 3 when compared to that of exemplary implementations 4 and 5.

As described above, from the results illustrated in FIG. 13 through FIG. 18, it was confirmed that the prediction accuracy of the material characteristics is improved by applying the S-shaped function, including the hyperbolic tangent function or the sigmoid function, as the activation function of the prediction model.

The embodiments are described above with reference to specific examples. However, the present disclosure is not limited to these specific examples. Those specific examples to which a person skilled in the art appropriately makes design modifications are also included in the scope of the present disclosure as long as the features of the present disclosure are included therein. Each element included in each specific example described above and the arrangement, condition, shape, or the like thereof are not limited to those illustrated, and can be appropriately modified. The elements included in the specific examples described above can be appropriately combined as long as no technical contradiction occurs.

The present international application is based on and claims priority to Japanese Patent Application No. 2021-043191, filed on March 17, 2021, and the entire contents of which are incorporated herein by reference.

### DESCRIPTION OF REFERENCE NUMERALS

10 Material characteristics prediction device
11 Trained model
11A Prediction model
12 Predicting part
20 Model generation device
21 Training data creating part
22 Model training part

## Claims

1. A material characteristics prediction method for predicting material characteristics of a target material, comprising:
model setting step of setting a trained model acquired by machine learning of a correspondence relationship between an explanatory variable including information related to a material composition or a manufacturing condition of the target material, and an objective variable including information related to the material characteristics of the target material; and
prediction step of inputting an explanatory variable related to a target material whose material characteristics is to be predicted to the trained model set in the model setting step, and outputting an objective variable related to information of the explanatory variable, so as to predict the material characteristics of the target material to be predicted based on the objective variable,
wherein the explanatory variable further includes information related to at least one of a material characteristics evaluation temperature that is a temperature at a time of measurement of the material characteristics included in the objective variable, and an evaluation temperature holding time that is a time during which the material characteristics evaluation temperature is held until the measurement of the material characteristics.

2. The material characteristics prediction method as claimed in claim 1, wherein the trained model is a neural network.

3. The material characteristics prediction method as claimed in claim 2, wherein
the material characteristics predicted in the prediction step are characteristics of a metal material, a polymer material, or a glass material that vary in an S-shape depending on the material characteristics evaluation temperature or the evaluation temperature holding time, and
an S-shaped function, including a hyperbolic tangent function or a sigmoid function, is used as an activation function of the neural network.

4. The material characteristics prediction method as claimed in claim 1, wherein a kernel method is applied as a machine learning method of the trained model.

5. The material characteristics prediction method as claimed in claim 4, wherein
the material characteristics predicted in the prediction step are characteristics of a metal material, a polymer material, or a glass material that vary in an S-shape depending on the material characteristics evaluation temperature or the evaluation temperature holding time, and
an inverse sine function is used as a kernel function of the kernel method.

6. A material characteristics prediction program for predicting material characteristics of a target material, the material characteristics prediction program causing a computer to implement:
a model setting function that sets a trained model acquired by machine learning of a correspondence relationship between an explanatory variable including information related to a material composition or a manufacturing condition of the target material, and an objective variable including information related to the material characteristics of the target material; and
a prediction function that inputs an explanatory variable related to a target material whose material characteristics is to be predicted to the trained model set in the model setting function, and outputs an objective variable related to information of the explanatory variable, so as to predict the material characteristics of the target material to be predicted based on the objective variable,
wherein the explanatory variable further includes information related to at least one of a material characteristics evaluation temperature that is a temperature at a time of measurement of the material characteristics included in the objective variable, and an evaluation temperature holding time that is a time during which the material characteristics evaluation temperature is held until the measurement of the material characteristics.

7. A material characteristics prediction device for predicting material characteristics of a target material, comprising:
a trained model acquired by machine learning of a correspondence relationship between an explanatory variable including information related to a material composition or a manufacturing condition of the target material, and an objective variable including information related to the material characteristics of the target material; and
a predicting part configured to input an explanatory variable related to a target material whose material characteristics is to be predicted to the trained model, and output an objective variable related to information of the explanatory variable, so as to predict the material characteristics of the target material to be predicted based on the objective variable,
wherein the explanatory variable further includes information related to at least one of a material characteristics evaluation temperature that is a temperature at a time of measurement of the material characteristics included in the objective variable, and an evaluation temperature holding time that is a time during which the material characteristics evaluation temperature is held until the measurement of the material characteristics.

8. A model generation method for generating a model for predicting material characteristics of a target material, the model generation method comprising:
training data creating step of acquiring a training data set including information related to a material composition or a manufacturing condition of the target material, material characteristics, and a measurement condition at a time when the material characteristics are measured; and
model training step of generating a trained model by performing machine learning so that an input-output relationship of the model approaches an input-output relationship of the training data set, using the training data set created in the training data creating step, by regarding the information related to the material composition or the manufacturing condition and the measurement condition as an input of the model, and information related to the material characteristics as an output of the model,
wherein the measurement condition includes at least one of a material characteristics evaluation temperature that is a temperature at a time of measurement of the material characteristics included in the output of the model, and an evaluation temperature holding time that is a time during which the material characteristics evaluation temperature is held until the measurement of the material characteristics.

9. A model generation program for generating a model for predicting material characteristics of a target material, the model generation program causing a computer to implement:
a training data creating function that acquires a training data set including information related to a material composition or a manufacturing condition of the target material, material characteristics, and a measurement condition at a time when the material characteristics are measured; and
a model training function that generates a trained model by performing machine learning so that an input-output relationship of the model approaches an input-output relationship of the training data set, using the training data set created in the training data creating function, by regarding the information related to the material composition or the manufacturing condition and the measurement condition as an input of the model, and information related to the material characteristics as an output of the model,
wherein the measurement condition includes at least one of a material characteristics evaluation temperature that is a temperature at a time of measurement of the material characteristics included in the output of the model, and an evaluation temperature holding time that is a time during which the material characteristics evaluation temperature is held until the measurement of the material characteristics.

10. A model generation device for generating a model for predicting material characteristics of a target material, the model generation device comprising:
a training data creating part configured to create a training data set including information related to a material composition or a manufacturing condition of the target material, material characteristics, and a measurement condition at a time when the material characteristics are measured; and
a model training part configured to generate a trained model by performing machine learning so that an input-output relationship of the model approaches an input-output relationship of the training data set, using the training data set created in the training data creating part, by regarding the information related to the material composition or the manufacturing condition and the measurement condition as an input of the model, and information related to the material characteristics as an output of the model,
wherein the measurement condition includes at least one of a material characteristics evaluation temperature that is a temperature at a time of measurement of the material characteristics included in the output of the model, and an evaluation temperature holding time that is a time during which the material characteristics evaluation temperature is held until the measurement of the material characteristics.
